Europäisches Patentamt

European Patent Office

Office européen des brevets

(11) Veröffentlichungsnummer: **0 113 808**
A2

# EUROPÄISCHE PATENTANMELDUNG

(21) Anmeldenummer: 83108530.3

(22) Anmeldetag: 30.08.83

(51) Int. Cl.³: **G 01 N 33/28**
// G01N27/62

(30) Priorität: 22.12.82 DD 246352

(43) Veröffentlichungstag der Anmeldung: 25.07.84
Patentblatt 84/30

(84) Benannte Vertragsstaaten: **AT BE CH DE FR GB IT LI SE**

(71) Anmelder: **VEB Transformatorenwerk "Karl Liebknecht", Wilhelminenhofstrasse 83-85, DDR-1160 Berlin (DD)**

(72) Erfinder: **Rindfleisch, Hans-Jochen, Dr. Ing., Salvador-Allende-Strasse 81, DDR-1170 Berlin (DD)**

(74) Vertreter: **Patentanwälte Beetz sen. - Beetz jun. Timpe - Siegfried - Schmitt-Fumian, Steinsdorfstrasse 10, D-8000 München 22 (DE)**

(54) Verfahren zur technischen Diagnose von ölgefüllten Hochspannungsgeräten.

(57) Dieses Verfahren eignet sich besonders zur Detektion und Charakterisierung von Schädigungsprozessen im technischen System von mit organischer Isolierflüssigkeit gefüllten Transformatoren, Wandlern und Drosselspulen mit Hilfe der in der Isolierflüssigkeit gelösten Gase (DGA). Ziel und Aufgabe der Erfindung bestehen darin, betriebsgefährdende elektrische und/oder thermische Anomalien im technischen System von Hochspannungsgeräten mit Feststoff-Flüssigkeitsisolierungen durch ein Verfahren für die kontinuierliche Betriebsüberwachung auf der Grundlage der DGA festzustellen und auszuwerten. Das wird dadurch erreicht, daß von den in der Isolierflüssigkeit gelösten Gasen die Summe der gasförmigen Kohlenwasserstoffe sowie des Sauerstoffs, des Wasserstoffs und des Kohlendioxids quantitativ bestimmt werden und mit den Konzentrationen dieser Gaskomponenten das Oxydationsmerkmal und das Rekombinationsmerkmal oder das Verkettungsmerkmal gebildet werden, und daß die Realisierungen des Oxydationsmerkmals einerseits und des Rekombinationsmerkmals oder des Verkettungsmerkmals anderseits bezüglich ihrer Korrelation nach Lage, Steigung und Reststreuung der Regressionsgeraden statistisch bewertet werden.

- 1 -

<u>Verfahren zur technischen Diagnose von ölgefüllten</u>
<u>Hochspannungsgeräten</u>

Die Erfindung bezieht sich auf ein Verfahren zur
technischen Diagnose von ölgefüllten Hochspannungsgeräten, insbesondere zur Detektion und Charakterisierung von Schädigungsprozessen im technischen
System von mit organischer Isolierflüssigkeit gefüllten Transformatoren, Wandlern und Drosselspulen mit Hilfe der in der Isolierflüssigkeit gelösten
Gase (DGA).

Hochspannungsgeräte haben häufig kombinierte Feststoff-Flüssigkeitsisolierungen, in denen die Flüssigkeit, ein synthetisches Öl oder ein Mineralöl,
sowohl als Isolier- und Tränkmittel als auch als
Kühlmittel dient. Diese kombinierten Flüssigkeits-
Feststoffisolierungen altern unter dem Einfluß der
gerätespezifischen elektrischen und thermischen
Beanspruchungen, wobei ein allmählicher chemischer
Abbau der festen und flüssigen Isolierstoffe stattfindet, der zum Verlust ihrer mechanischen Eigenschaften beziehungsweise Isolier- und Kühleigenschaften und schließlich zum Ausfall wesentlicher
Funktionen des Hochspannungsgerätes führt, was häufig mit folgeschweren Havarien verbunden ist. Auf der Grundlage langjähriger Betriebserfahrungen und gezielter Langzeitversuche mit derartigen Isoliersystemen werden die mit ihnen ausgestatteten Hochspannungsgeräte so gestaltet und bemessen, daß die in ihnen bei Betrieb dauernd auftretenden Temperaturen und elektrischen Feldstärken bestimmte, für den jeweiligen Gerätetyp maximal zulässige Grenzen nicht überschreiten, so daß eine Lebensdauer des Gerätes in der volkswirtschaftlich erforderlichen Höhe gewährleistet ist. Neben den projektierten Beanspruchungen der festen und flüssigen Isolierstoffe, die für die normale Alterung des Isoliersystems bestimmend sind, treten in Hochspannungsgeräten nicht selten auch bedeutend höhere thermische und/oder elektrische Beanspruchungen auf, die örtlich begrenzt zu einer beschleunigten, anormalen Alterung des Isoliersystems führen. Solche elektrischen und/oder thermischen Anomalien im Isoliersystem eines Hochspannungsgerätes entstehen in der Regel infolge von Mängeln in der Konstruktion, bei der Fertigung oder Inbetriebnahme des Gerätes, können aber auch durch zeitweise starke elektrische, dynamische oder thermische Überlastungen des Hochspannungsgerätes während seines Betriebes hervorgerufen werden. Abhängig vom Ort ihres Auftretens im technischen System des Hochspannungsgerätes und der dort vorhandenen Redundanz, dem Grad der durch sie hervorgerufenen Funktionsbeeinträchtigung und ihrem "Selbstheilungsvermögen" hat eine thermische oder elektrische Anomalie bei jeweils gleicher Beanspruchung der Isolierstoffe sehr unterschiedliche Auswirkungen auf die Betriebssicherheit und die Lebensdauer eines Hochspannungsgerätes.

- 3 -

So kann zum Beispiel eine thermische Anomalie in einer Transformatorenwicklung durch einen erhöhten Übergangswiderstand infolge mangelhafter Lötung an der Verbindungsstelle zweier Stromleiter hervorgerufen werden. Die im erhöhten Übergangswiderstand entstehenden Stromwärmeverluste verursachen an dieser Stelle Temperaturen, die weit über den dauernd zulässigen Grenzwerten liegen und daher dort zu einer beschleunigten Alterung der Isolation führen. Setzt sich die Isolierstrecke zwischen dem betroffenen Stromleiter und benachbarten Bauteilen unterschiedlichen Potentials aus einem festen und einem flüssigen Anteil zusammen, so führt die thermische Anomalie zwar zu einer vorzeitigen Zerstörung des festen Anteils, aber nicht zum völligen Verlust der Isolationsfestigkeit dieser Strecke, da sich ihr flüssiger Anteil bedingt durch konvektiven Stoffaustausch ständig regenerieren kann. In einem solchen Falle liegt zwar eine beschleunigte Alterung, jedoch ein stabiles Alterungsverhalten vor, wenn sich der anormale Alterungsprozeß räumlich nicht ausdehnt und seinen chemisch-physikalischen Mechanismus beibehält, so daß er sich in seiner Auswirkung auf die Betriebstauglichkeit des Hochspannungsgerätes nicht ändert. Im vorliegenden Beispiel kann der anormale Alterungsprozeß infolge Schwächung der Isolationsfestigkeit zwar zu einer potentiellen Gefährdung der Betriebssicherheit des Hochspannungsgerätes, insbesondere durch gelegentlich auftretende Überspannungen, jedoch nicht zu einer akuten Betriebsgefährdung führen, da die mindestens verbleibende Isolationsfestigkeit ausreicht, um den ständig auftretenden elektrischen Beanspruchungen standzuhalten. Enthält die Isolierstrecke da-

- 4 -

gegen nur festen Isolierstoff, so führt die thermische Anomalie frühzeitig zu einem völligen Ausfall der Isolation dieser Strecke. Hierbei ändert der Alterungsprozeß infolge Auftretens elektrischer Entladungen nicht nur seinen chemisch-physikalischen Mechanismus sondern dehnt sich infolge der durch die Entladungen hervorgerufenen Kurzschlußströme auch weiter in die Wicklung aus, wobei er außerdem an Intensität zunimmt. In einem solchen Falle liegt ein instabiles Alterungsverhalten vor, das durch zunehmend beschleunigte Zerstörung von Teilen der Isolation oder anderer funktionswesentlicher Bauelemente infolge anomaliebedingt zunehmender elektrischer und/oder thermischer Beanspruchungen in einem sich räumlich ausdehnenden Anomaliegebiet gekennzeichnet ist. Ein solches Alterungsverhalten ruft naturgemäß eine akute Betriebsgefährdung des Hochspannungsgerätes hervor, da es in den meisten Fällen zu einem völligen Versagen seiner Funktionsfähigkeit führt.

Für die Beurteilung der Betriebszuverlässigkeit eines Hochspannungsgerätes ist somit nicht allein die Feststellung der Existenz einer Anomalie in der Alterung seines Isoliersystems und der Art und Intensität dieser Anomalie, wie die Höhe der Temperatur an einer Heißstelle oder die Energiedichte von elektrischen Entladungen, sondern vor allem die Kenntnis des durch sie bedingten Alterungsverhaltens erforderlich.

Es ist bekannt, die im Alterungsprozeß der festen und flüssigen Isolierstoffe eines Isoliersystems infolge thermischer und/oder elektrischer Beanspruchung entstehenden und im flüssigen Isoliermedium gelösten gasförmigen Abbauprodukte nach Art und Konzentration zu bestimmen, um daraus Aufschluß über den Alterungsprozeß zu bekommen. Ausgehend von der Vorstellung, daß diese Abbauprodukte ausschließlich durch pyrolytische Spaltung entstehen, wird entweder aus dem Vorhandensein bestimmter kennzeichnender Spaltprodukte oder aus dem Verhältnis ihrer Konzentrationen auf die Existenz, Art und Intensität von Anomalien geschlossen. Die Merkmale gasanalytischer Befunde werden jeweils in Wertebereiche aufgeteilt und kodiert, wobei den gasanalytischen Befunden von typischen Anomalien bestimmte Zahlenkombinationen zugeordnet sind.

Es hat sich gezeigt, daß diese auf eine beschränkte Zahl empirischer Ergebnisse, insbesondere der Auswertung von Schadens- und Revisionsbefunden beruhenden Codeschemata von nur begrenzter Gültigkeit sind und häufig für gasanalytische Befunde keine Aussage liefern. Ein besonderer Nachteil dieses Verfahrens besteht darin, daß es keinen Aufschluß über das Alterungsverhalten und damit über den durch eine Anomalie bedingten Gefährdungsgrad eines Hochspannungsgerätes geben kann. Daher ist es auch üblich, die zeitliche Veränderung der Spaltproduktkonzentrationen zu bestimmen, um rechtzeitig ein betriebsgefährdendes Alterungsverhalten zu erkennen. Es hat sich aber herausgestellt, daß die zeitlichen Veränderungen der Spaltproduktkonzentrationen nach einiger Betriebszeit den Charakter statistischer Schwankungen haben, die durch die Thermodynamik des Alterungsprozesses und durch Belastungsschwankungen sowie zufällig auftretende, vorübergehende Überlastungen hervorgerufen werden. Da diese Einflußgrößen einer statistischen Erfassung und Auswertung kaum zugänglich und daher auch korrelativ nicht eliminierbar sind, kann eine signifikante zeitliche Korrelation der Spaltproduktkonzentrationen und damit eine Anomalie mit instabiler Alterungscharakteristik erst erkannt werden, wenn sie bereits ein unmittelbar betriebsgefährdendes Entwicklungsstadium erreicht hat.

Da das Diagnoseverfahren auf der Entnahme von Proben der Isolierflüssigkeit aus dem Hochspannungsgerät und ihrer anschließenden Analyse in einem in der Regel nicht am gleichen Ort befindlichen Labor beruht, kann die Überwachung mit Hilfe dieses Verfahrens nur diskontinuierlich, das heißt in bestimmten Zeitabständen erfolgen. Diese Zeitabstände so kurz zu wählen, daß anhand der zeitlichen Veränderung der Spaltproduktkonzentrationen der kritische Zustand einer Anomalie und damit eine akute Gefährdung des Hochspannungsgerätes sicher und rechtzeitig erkannt werden kann, ist mit einem sehr hohen Aufwand verbunden und daher nicht generell, sondern nur in besonderen Fällen möglich.

Es ist daher bereits bekannt, Hochspannungsgeräte kontinuierlich mit Hilfe automatisch arbeitender Analysengeräte gasanalytisch zu überwachen. Hierbei wird nur eine Gaskomponente, in der Regel der Wasserstoff, analytisch bestimmt und bei Überschreiten eines Grenzwertes der Konzentration oder der Konzentrationsänderung ein betriebsgefährdender Anomaliezustand angezeigt. Der Nachteil dieses Verfahrens liegt auf der Hand, denn betriebsgefährdende Anomalien lassen sich aufgrund sehr unterschiedlicher Spezifik naturgemäß nicht nur an einer Gaskomponente gasanalytisch rechtzeitig und sicher erkennen.

Daher sind auch Gasanalysatoren bekannt geworden, die eine Analyse aller im Öl gelösten Gase ausführen. Der Nachteil dieser automatischen Gasanalysatoren besteht in einem erheblichen Aufwand sowohl für den apparativen Aufbau als auch für die erforderliche Wartung und Instandhaltung, so daß sich ihre generelle Anwendung im Dauerbetrieb von Hochspannungsgeräten bisher als nicht zweckmäßig herausgestellt hat.

Beiden Verfahren zur kontinuierlichen gasanalytischen Überwachung von ölgefüllten Hochspannungsgeräten haftet überdies der Mangel an, daß mit ihrer Hilfe aufgrund bloßer Erfassung von Gaskonzentrationen und/oder ihrer zeitlichen Veränderungen eine wirklich betriebsgefährdende Anomalie erst sehr spät erkannt werden kann. Daher erlauben diese Verfahren allenfalls nur, folgenschweren Havarien vorzubeugen. Sie erlauben nicht, Reservebereitstellung und Reparatur mittelfristig zu planen und so Ausfälle und Verluste bei der Energieerzeugung und Energieübertragung zu vermeiden.

- 8 -

Ziel der Erfindung ist es, betriebsgefährdende elektrische und/oder thermische Anomalien im technischen System von ölisolierten Hochspannungsgeräten mit angemessenem Aufwand rechtzeitig und sicher zu erkennen, um folgeschweren Havarien wirksam vorbeugen sowie Art und erforderlichen Umfang der Reservebereitstellung und Instandsetzungsmaßnahmen in einem für ihre Planung und Vorbereitung erforderlichem Zeitraum im voraus bestimmen zu können.

Der Erfindung liegt die Aufgabe zugrunde, ein für die kontinuierliche Betriebsüberwachung von Hochspannungsgeräten mit Feststoff-Flüssigkeitsisolierungen geeignetes Verfahren zu schaffen, das auf der quantitativen Bestimmung der in der Isolierflüssigkeit gelösten gasförmigen Spaltprodukte und der statistischen Bewertung der entsprechenden gasanalytischen Befunde beruht, und das es gestattet, diejenigen elektrischen und/oder thermischen Anomalien, die mit hoher Wahrscheinlichkeit eine Betriebsgefährdung des Hochspannungsgerätes hervorrufen können, von denjenigen zu unterscheiden, die die Betriebssicherheit des Hochspannungsgerätes mit hoher Wahrscheinlichkeit nicht beeinträchtigen, und dabei den erforderlichen Grad der gasanalytischen Differenzierung bei der Bestimmung der Spaltprodukte zu verringern sowie auf die statistische Erfassung und Bewertung der Belastungen und sonstigen Betriebsbedingungen des Hochspannungsgerätes weitgehend zu verzichten.
Erfindungsgemäß wird dies dadurch erreicht, daß von den in der Isolierflüssigkeit gelösten Gasen die Summe der bei Raumtemperatur und Normaldruck gasförmigen Kohlenwasserstoffe, mindestens bis einschließlich den Kohlenwasserstoffen mit drei Kohlenstoffatomen $\sum_{3} C_n H_m$ , das Kohlendioxid $CO_2$, der Sauerstoff $O_2$ und der Wasserstoff $H_2$ quantitativ bestimmt werden, und daß mit den Konzentrationen dieser Gaskomponenten das Oxydationsmerkmal

$$\log \frac{\sum_{3}[C_n H_m] \cdot [O_2]}{[H_2] \cdot [CO_2]} \qquad \text{und das Rekombinations-merkmal}$$

$$\log \frac{\sum_{3}[C_n H_m]}{[H_2] + [CO_2]} \qquad \text{oder das Verkettungs-merkmal}$$

$$\log \left( \frac{[O_2]}{[H_2]} + \frac{[O_2]}{[CO_2]} \right) \qquad \text{gebildet werden, und}$$

daß die Realisierungen des Oxydationsmerkmals einerseits und des Rekombinationsmerkmals oder des Verkettungsmerkmals andererseits bezüglich ihrer Korrelation nach Lage, Steigung und Reststreuung der Regressionsgeraden statistisch bewertet werden dergestalt, daß typdeterminierte Regressionsgeraden von Hochspannungsgeräten einerseits und eine fehlerdeterminierte Regressionsgerade von betriebsgefährdenden Anomalien andererseits erhalten werden.

Anstelle der Summe der Kohlenwasserstoffkonzentrationen kann auch die entsprechende molare Konzentration des Kohlenstoffs $\left[ C \cdot \left( \sum_{3} C_n H_m \right) \right]$ für die Bestimmung der gasanalytischen Merkmale verwendet werden. Es hat sich überraschend herausgestellt, daß die erfindungsgemäß bestimmten Merkmale gasanalytischer Befunde trotz großer Unterschiede in den konstruktiven Eigenarten von Hochspannungsgeräten und in den Schädigungsmerkmalen von Anomalien eine für alle betriebsgefährdenden thermischen und/oder elektrischen Anomalien gemeinsame Korrelation aufweisen. Dadurch ist es möglich, unabhängig von Konstruktionsart des Gerätes und Schädigungsart der Anomalie ein allgemeingültiges Grenzgebiet der Schädigung durch eine einzige Regressionsgerade und ihre Reststreuung statistisch darzustellen, innerhalb dessen sich der Übergang von beginnender potentieller Gefährdung zur unmittelbaren akuten Betriebsgefährdung vollzieht.

Weiterhin hat sich erwiesen, daß die erfindungsgemäß gebildeten gasanalytischen Merkmale aller Hochspannungsgeräte eines Konstruktionstyps mit normalem, nicht fehlerdeterminiertem Alterungsverhalten in weiten Grenzen unabhängig von Belastung und sonstigen Betriebsbedingungen eine gemeinsame Korrelation zeigen. Dadurch ist es möglich, einen für alle Geräte eines Konstruktionstyps gültigen Bereich durch eine Regressionsgerade und ihre Reststreuung darzustellen, innerhalb dessen sich weitgehend unabhängig von betriebsbedingten Einflußgrößen die für normales Alterungsverhalten kennzeichnenden gasanalytischen Befunde aufhalten. Durch die Lage, Steigung und Reststreuung der typdeterminierten Regressionsgeraden in Bezug auf die fehlerdeterminierte Regressionsgerade betriebsgefährdender Anomalien kann festgestellt werden, ob konstruktiv bedingte Anomalien vorliegen, die das typspezifische Alterungsverhalten bestimmen. Durch die Lage eines oder erforderlichenfalls mehrerer zeitlich nacheinander aufgenommener gasanalytischer Befunde in Bezug auf die entsprechende typdeterminierte Regressionsgerade kann festgestellt werden, ob das betreffende Gerät sich im Rahmen typspezifischen Alterungsverhaltens bewegt oder ob bereits Anzeichen fehlerdeterminierten Alterungsverhaltens aufgrund einer exemplarischen Anomalie vorliegt, die durch Fertigungs- oder Materialfehler oder auch durch extreme Betriebsbedingungen hervorgerufen sein kann. Durch die Bestimmung der erfindungsgemäßen gasanalytischen Merkmale und ihre statistische Bewertung wird somit erreicht, daß allein schon die durch ein Wertepaar dieser Merkmale bestimmte Lage des gasanalytischen Befundes eines Hochspannungsgerätes bezüglich seiner typdeterminierten Regressionsgeraden bzw. der fehlerdeterminierten Regressionsgerade betriebsgefährdender Anomalien eine mit hoher Wahrscheinlichkeit zutreffende Aussage zur Betriebssicherheit dieses Hochspannungsgerätes zuläßt, ohne daß hierzu zusätzliche Informationen über die Betriebsbedingungen erforderlich sind.

Damit werden Voraussagen über die Betriebszuverlässigkeit des Hochspannungsgerätes sowie erforderliche vorbeugende Instandhaltungs- und Überwachungsmaßnahmen für einen zu deren Planung, Vorbereitung und Durchführung erforderlichen Zeitraum möglich. Das bedeutet auch, daß der Betrieb eines Hochspannungsgerätes bis an seine Schädigungsgrenze möglich ist, was seine Verfügbarkeit erhöht und damit eine geringere Reservehaltung erforderlich macht.

Darüber hinaus eignen sich die für die Bildung der erfindungsgemäßen gasanalytischen Merkmale erforderlichen Stoffkomponenten aufgrund ihrer sehr unterschiedlichen chemischen und physikalischen Eigenschaften besonders für eine automatische Detektion, ohne daß hierzu eine gaschromatographische Auftrennung des Gasgemisches in seine Komponenten
erforderlich ist. Dadurch ist es möglich, auf
die Verwendung des zwar sehr leistungsfähigen,
aber störanfälligen und schon allein wegen des
Bedarfs ständig neu aufzufüllender Gasreservoirs
wartungsaufwendigen Gas-Chromatographen zu verzichten und ein automatisch arbeitendes gasanalytisches Überwachungssystem mit einfachen, komponentenspezifischen Detektoren aufzubauen, die
wartungsarm und wenig störanfällig sind.

Anhand eines Beispiels soll die Erfindung näher
erläutert werden.

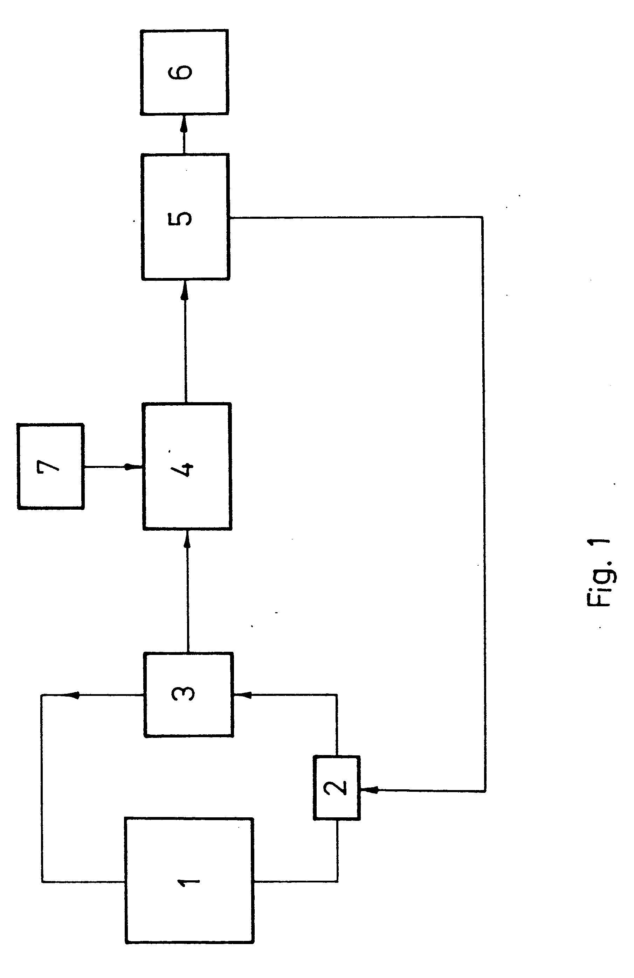

In Figur 1 ist das Blockschaltbild einer automatischen Überwachungseinrichtung für ölgefüllte
Hochspannungsgeräte gezeigt, welche die für eine
technische Diagnose nach dem erfindungsgemäßen
Verfahren erforderlichen Funktionseinheiten enthält.

Das gashaltige Isolieröl wird aus dem Hochspannungsgerät 1 durch eine automatische Probennahmevorrichtung 2 entnommen und in die Entgasungsapparatur 3 geleitet. Dort wird das Öl entgast
und danach wieder dem Hochspannungsgerät zugeführt. Die aus dem Öl abgetrennten gasförmigen
Komponenten gelangen in ein Massenspektrometer 4,
mit Hilfe dessen das Massenspektrogramm der Gasprobe erzeugt wird, aus dem vorzugsweise die Molekülpeaks der in der Gasprobe enthaltenen Gaskomponenten zu ihrer quantitativen Bestimmung als
Eingangssignale einem Mikrorechner 5 zugeleitet
werden. Mit Hilfe der Matrix der Eichwerte $\varepsilon_{i_k}$

und des Spaltenvektors der gemessenen Intensitätswerte $\mathcal{L}_k$ werden von dem Mikrorechner 5 die Partialdrücke pi der einzelnen Gaskomponenten im Gasgemisch und daraus die erfindungsgemäßen gasanalytischen Merkmale der Gasprobe bestimmt. Danach führt der Mikrorechner 5 die Bewertung der gasanalytischen Merkmale nach dem erfindungsgemäßen Verfahren aus und gibt das Ergebnis in Form von Gefährdrungsstufen aus. Über ein Signalsystem 6 wird die erreichte Gefährdungsstufe ausgewiesen. Die Überwachung wird vom Rechner abhängig vom Analyseergebnis automatisch gesteuert. Zur Kontrolle und gegebenenfalls Korrektor der Eichwerte $\mathcal{E}i_k$ ist ein Eichgasbehälter 7 vorgesehen, aus dem in bestimmten Zeitabständen Eichgasproben dem Massenspektrometer zugeführt werden. Benutzt man hierzu ein Flüssiggas, zum Beispiel Butan, so kann eine für die Betriebsdauer des Hochspannungsgerätes ausreichende Menge an Eichgas in dem Eichgasbehälter 7 untergebracht werden.

Für die Entgasung der Ölprobe in der Entgasungsapparatur 3 wird zweckmäßig das Vorvakuum des Massenspektrometers 4 genutzt. Das Massenspektrometer ist vorzugsweise mit einer Einrichtung zur Feldionisation ausgerüstet, um eine Fragmentierung der in der Gasprobe enthaltenen Gaskomponenten zugunsten der Bildung von Molekülionen zurückzudrängen.

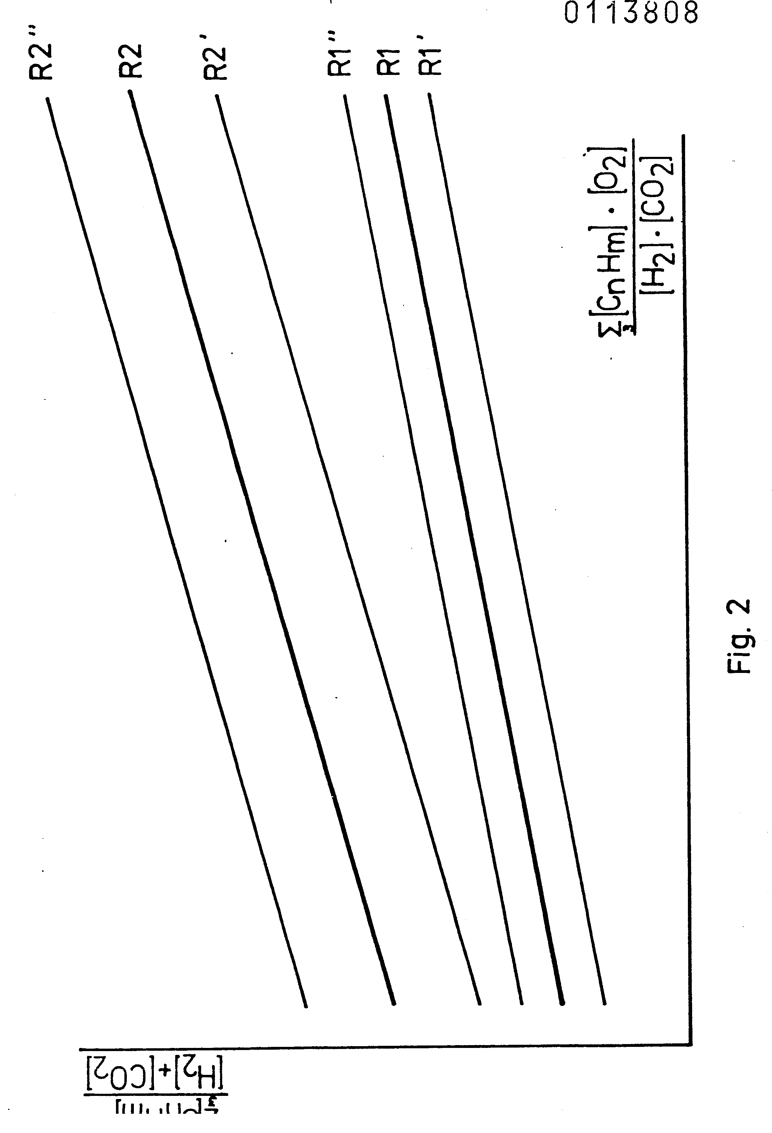

In der Figur 2 ist das erfindungsgemäße Verfahren der technischen Diagnose von ölgefüllten Hochspannungsgeräten graphisch dargestellt. An der Abszisse ist das Oxidationsmerkmal

$$\log \frac{\sum[\ ] \cdot [\ ]}{[\ ] \cdot [\ ]}$$

und an der Ordinate das Rekombinationsmerkmal

$$\log \frac{\sum [C_i H_m]}{[H_2] + [CO_2]}$$

aufgetragen. Es hat sich überraschend gezeigt, daß diese erfindungsgemäßen gasanalytischen Merkmale für jeden Typ von Hochspannungsgerät eine spezifische lineare Korrelation entsprechend der Regressionsgeraden R1 ergeben.

Weiterhin hat sich gezeigt, daß alle zu Betriebsstörungen führenden elektrischen und/oder thermischen Anomalien, also Schädigungsprozesse, die die Schädigungsgrenze erreicht haben, unabhängig von der Art des Schädigungsprozesses und dem Konstruktionstyp des Hochspannungsgerätes erfindungsgemäße gasanalytische Merkmale hervorrufen, die eine signifikante Korrelation entsprechend der Regressionsgeraden R2 ergeben.

Befindet sich nun ein gasanalytischer Befund in dem durch die 5 %- und 95 %-Linien R1' bzw. R1'' zur Regressionsgeraden R1 begrenzten typspezifischen Bereich des betreffenden Hochspannungsgerätes, so liegt ein ungestörtes Betriebsverhalten vor (Gefährdungsstufe 0). Liegt der gasanalytische Befund im Grenzgebiet zwischen der 95 %-Linie R1'' zur Regressionsgeraden R1 und der 5 %-Linie R2' zur Regressionsgeraden R2, so liegen erste Anzeichen einer potentiellen Betriebsgefährdung (Gefährdungsstufe 1) vor. Eine strengere gasanalytische Überwachung und eine langfristige Planung von Instandhaltungsmaßnahmen sind dann erforderlich. Hat der gasanalytische Befund das Gebiet zwischen der 5 %-Linie R2' zur Regressionsgeraden R2 und der Regressionsgeraden R2 erreicht, so ist die Wahrscheinlichkeit für das Eintreten einer Betriebsstörung, insbesondere bei kurzzeitigen Überlastungen des Hochspannungsgerätes, nicht mehr zu vernachlässigen. Es liegt dann eine potentielle Betriebsgefährdung vor (Gefährdungsstufe 2). Eine eingehende Überprüfung des Hochspannungsgerätes vor Ort und seiner Schutzeinrichtungen sowie eine mittelfristige Planung von Instandhaltungsmaßnahmen sind dann erforderlich. Gelangt der gasanalytische Befund in das Gebiet zwischen der Regressionsgeraden R2 und der ihr zugehörigen 95%-Linie R2'', so ist mit einer hohen Wahrscheinlichkeit der Eintritt einer Betriebsstörung des Hochspannungsgerätes auch während des normalen Betriebs zu erwarten. In diesem Fall ist der Tatbestand der akuten Betriebsgefährdung gegeben (Gefährdungsstufe 3), der eine kurzfristige Außerbetriebnahme zwecks Instandsetzung im Reparaturwerk erforderlich macht.

Die Regressionsgerade R2 ist somit unabhängig von der Art des Schädigungsprozesses als generelle Schädigungsgrenze zu betrachten. Ihre Kenntnis macht es möglich, die tatsächliche Verfügbarkeit eines ölgefüllten Hochspannungsgerätes mit Hilfe der gasanalytischen Überwachung maximal zu nutzen. Die 5%- und 95%-Linien R2' und R2'' erlauben es, das dabei auftretende Risiko quantitativ abzuschätzen.

Patentansprüche

1. Verfahren zur Detektion und Charakterisierung von Schädigungsprozessen (technische Diagnose) im technischen System von mit organischer Isolierflüssigkeit gefüllten Hochspannungsgeräten mit Hilfe der in der Isolierflüssigkeit gelösten Gase (DGA), gekennzeichnet dadurch, daß von den in der Isolierflüssigkeit gelösten Gasen die Summe der bei Raumtemperatur und Normaldruck gasförmigen Kohlenwasserstoffe, mindestens bis einschließlich den Kohlenwasserstoffen mit drei Kohlenstoffatomen $\sum_3 C_n H_m$, das Kohlendioxid $CO_2$, der Sauerstoff $O_2$ und der Wasserstoff $H_2$ quantitativ bestimmt werden, und daß mit den Konzentrationen dieser Gaskomponenten das Oxidationsmerkmal

$$\log \frac{\sum_3 [C_n H_m] \cdot [O_2]}{[H_2] \cdot [CO_2]}$$

und das Rekombinationsmerkmal

$$\log \frac{\sum_3 [C_n H_m]}{[H_2] + [CO_2]}$$

oder das Verkettungsmerkmal

$$\log \left( \frac{[O_2]}{[H_2]} + \frac{[O_2]}{[CO_2]} \right)$$

gebildet werden, und daß die Realisierungen des Oxidationsmerkmals einerseits und des Rekombinationsmerkmals oder des Verkettungsmerkmals andererseits bezüglich ihrer Korrelation nach Lage, Steigung und Reststreuung der Regressionsgeraden statistisch bewertet werden dergestalt, daß typdeterminierte Regressionsgeraden ungestörten Betriebsverhaltens von Hochspannungsgeräten und eine fehlerdeterminierte Regressionsgerade von betriebsgefährdenden Schädigungsprozessen erhalten werden, und daß die Lage eines durch die Größen von Oxidationsmerkmal und Rekombinations- bzw. Verkettungsmerkmal gekennzeichneten Befundes in Bezug auf die typdeterminierte Regressionsgerade ungestörten Betriebsverhaltens und die fehlerdeterminierte Regressionsgerade betriebsgefährdender Schädigungsprozesse zur Bestimmung der Betriebssicherheit und Verfügbarkeit des Hochspannungsgerätes mit Hilfe geeigneter statistischer Methoden genutzt wird.

2. Verfahren nach Patentanspruch 1, gekennzeichnet dadurch, daß anstelle der Summe der Kohlenwasserstoffkonzentrationen $\Sigma[C_nH_m]$ die entsprechende molare Konzentration des Kohlenstoffs $[\Sigma(\Sigma c_n H_m)]$ bestimmt und für die technische Diagnose genutzt wird.

3. Verfahren nach Patentanspruch 1 und 2, gekennzeichnet dadurch, daß für die quantitative Bestimmung der Konzentrationen der Gaskomponenten Analysengeräte und Detektoren, zum Beispiel Massenspektrograph, verwendet werden, die keine Reservoirs für Betriebsstoffe mit zeitlich begrenzter Kapazität benötigen.

4. Verfahren nach Patentanspruch 1 bis 3, gekennzeichnet dadurch, daß die Ausgangssignale des Analysengerätes on line einem Mikrorechner zur quantitativen Bestimmung der Gaskonzentration und gasanalytischen Merkmale, zur statistischen Bewertung und zur Signalisierung von Gefährdungsstufen übertragen werden.

Fig. 1

Fig. 2